# EUROPÄISCHE PATENTSCHRIFT

(11) **EP 3 146 920 B1**
(45) Veröffentlichungstag und Bekanntmachung des Hinweises auf die Patenterteilung: **15.11.2017**
(21) Anmeldenummer: 15186573.0
(22) Anmeldetag: 23.09.2015
(51) Int. Cl.: A61B 17/3203, A61B 90/98

(54) **VORRICHTUNG ZUR ERZEUGUNG EINES FLÜSSIGKEITSSTRAHLS**
DEVICE FOR PRODUCING A FLUID JET
DISPOSITIF DESTINE A LA PRODUCTION D'UN JET DE LIQUIDE

(43) Veröffentlichungstag der Anmeldung: 29.03.2017
(73) Patentinhaber: MEDAXIS AG, 6340 Baar (CH)
(72) Erfinder: Moser, Beat, 8926 Uerzlikon (CH); Zweifel, Adrian, 8645 Jona (CH); Widmer, Beat, 6005 Luzern (CH); Bütler, Martin, 6276 Hohenrain (CH); Christen, Lukas, 6004 Luzern (CH); Good, Roman, 8057 Zürich (CH); Napoletano, Daniel, 8193 Eglisau (CH)
(74) Vertreter: Grünecker Patent- und Rechtsanwälte PartG mbB

(56) Entgegenhaltungen:
- US-A1- 2001 002 562
- US-A1- 2002 176 788
- US-A1- 2008 221 602
- US-A1- 2010 049 228
- US-A1- 2011 150 680
- US-A1- 2014 079 580

## Beschreibung

Die vorliegende Erfindung betrifft eine Vorrichtung zur Erzeugung eines Flüssigkeitsstrahls, insbesondere für die Entfernung von biologischem Gewebe.

Aus der EP 1 924 305 B1, WO 2007/031304 A1, der US 7,553,318 B2 bzw. der WO 01/97700 A2 sind Vorrichtungen zur Erzeugung eines Wasserstrahls für die Behandlung von biologischem Gewebe bekannt. Dabei geht es der vorliegenden Erfindung insbesondere um das Debridement mittels Wasserstrahl. Beim Debridement wird zur Förderung der Wundheilung die Wunde gereinigt, üblicherweise werden auch Schorf und Überwachsungen entfernt. Die zuvor erwähnten Druckschriften belegen das Bemühen der Fachwelt, Lösungen vorzuschlagen, dieses Debridement mittels Wasserstrahl durchzuführen. Ein weiterer Beleg, der auf die vorliegende Anmelderin zurückgeht, ist durch die EP 1 296 601 B1 gegeben.

Bei Gerätschaften, die im Krankenhaus und insbesondere bei der mechanischen Behandlung von Wunden zum Einsatz kommen, besteht das Bedürfnis, gewisse bei der Behandlung mittels Flüssigkeitsstrahl zum Einsatz kommende Komponenten als Verbauchsmaterial oder zumindest sterilisierbar auszubilden. Andererseits lassen sich gewisse Bauteile einer Vorrichtung zur Erzeugung eines Flüssigkeitsstrahles, beispielsweise zumindest der elektrische Antrieb einer Pumpe nicht effektiv sterilisieren und sind auch regelmäßig zu kostspielig, um als Wegwerfprodukte ausgebildet zu werden.

Eine Vorrichtung zur Erzeugung eines Flüssigkeitsstrahls für medizinische und chirurgische Anwendungen gemäß den oberbegrifflichen Merkmalen von Anspruch 1 ist aus der US 2002/0176788 A1 und aus der US 2010/0049228 A1 bekannt. Dieser Stand der Technik offenbart auch eine Antriebseinheit zur Erzeugung eines Flüssigkeitsstrahls gemäß den oberbegrifflichen Merkmalen von Anspruch 13 und ein Pumpenmodul gemäß den Oberbegrifflichen Merkmalen von Anspruch 14.

Die vorliegende Erfindung will eine Vorrichtung zur Erzeugung eines Flüssigkeitsstrahls angeben, die sich wirtschaftlich und einfach herstellen lässt und den oben genannten Anforderungen genügt. Des Weiteren will die vorliegende Erfindung eine verbesserte Antriebseinheit und ein verbessertes Pumpenmodul einer solchen Vorrichtung zur Erzeugung eines Flüssigkeitsstrahls angeben.

Zur Lösung dieses Problems schlägt die vorliegende Erfindung eine Vorrichtung mit den Merkmalen von Anspruch 1, eine Antriebseinheit mit der Merkmalen von Anspruch 13 und ein Pumpenmodul mit der Merkmalen von Anspruch 14 vor. Die erfindungsgemäße Vorrichtung hat ein Antriebsgehäuse, in welchem ein Antrieb vorgesehen ist. Hierbei handelt es sich üblicherweise um einen elektrisch angetriebenen Antrieb. Das Antriebsgehäuse nimmt diesen elektrischen Antrieb in sich auf und weist üblicherweise Bedienelemente für das An- und Ausschalten sowie das Steuern des Antriebes auf. Des Weiteren hat die erfindungsgemäße Vorrichtung ein Pumpenmodul. Dieses Pumpenmodul umfasst ein Pumpengehäuse sowie üblicherweise Mittel innerhalb des Pumpengehäuses, die dazu geeignet sind, ein dem Pumpengehäuse zugeführtes Fluid unter Druck zu setzen und zu fördern. Das Pumpengehäuse kann beispielsweise zumindest einen Kolben aufweisen, der mit Ein- und Auslassöffnungen für das Fluid kommuniziert, um dieses Fluid an der Einlassöffnung anzusaugen und an der Auslassöffnung mit erhöhtem Druck abzugeben. Zur gerichteten Förderung des Fluides kann das Pumpengehäuse Ventile aufweisen. Das Pumpengehäuse an sich kann einen relativ einfachen Aufbau aufweisen und als Einwegteil ausgebildet sein, insbesondere als Kunststoffteil. Bevorzugt sind sämtliche Komponenten des Pumpenmoduls aus Kunststoff gebildet.

So wird mit der vorliegenden Erfindung eine modulare Ausgestaltung der Vorrichtung vorgeschlagen, bei welcher das Pumpengehäuse einerseits und das Antriebsgehäuse andererseits lösbar zu fügen sind.

Erfindungsgemäß hat das Antriebsgehäuse zumindest ein Formschlusselement. Das Pumpengehäuse hat ein mit diesem Formschlusselement beim Fügen von Pumpen- und Antriebsgehäuse zusammenwirkendes Formschlussgegenelement. Das Formschlusselement und das Formschlussgegenelement wirken dabei bis zum Erreichen einer Endposition so zusammen, dass dem Pumpengehäuse eine Schwenkbewegung relativ zu dem Antriebsgehäuse aufgeprägt wird, bis das Pumpengehäuse seine Endposition erreicht hat. Die Endposition des Pumpengehäuses ist diejenige Position, in der das Pumpengehäuse mit dem Antriebsgehäuse verbunden ist und zumindest ein an dem Antriebsgehäuse vorgesehenes Antriebselement des Antriebs0 mit einem zugeordneten Antriebsgegenelement des Pumpenmoduls verbunden ist. Dabei sind das Antriebselement und das Antriebsgegenelement exzentrisch zu einer Schwenkachse einer Schwenkbewegung angeordnet, die beim Fügen von Antrieb und Pumpenmodul dem Pumpenmodul relativ zu dem Antriebsgehäuse aufgeprägt wird. Die Ausgestaltung von Antriebselement und Antriebsgegenelement ist derart, dass sich aufgrund der aufgezwungenen Schwenkbewegung bis zum Erreichen der Endposition eine formschlüssige Verbindung zwischen Antriebselement und Antriebsgegenelement ergibt.

So wird mit der vorliegenden Erfindung sichergestellt, dass das Fügen von Pumpenmodul und Antrieb zwangsläufig zu einer formschlüssigen Verbindung der jeweiligen Elemente zum Antrieb der Pumpe führt. Dabei ist das Antriebselement mit dem Antrieb gekoppelt, wohingegen das Antriebsgegenelement regelmäßig unmittelbar oder mittelbar mit einem Kolben verbunden ist, der das Fluid in einem Zylinder des Pumpenmoduls unter Druck setzt.

Die erfindungsgemäße Vorrichtung lässt sich somit einfach handhaben. Auch ohne technischen Sachverstand führt das Fügen von Antriebsgehäuse und Pumpenmodul dazu, dass die beiden Antriebselemente der Vorrichtung antriebsmäßig zwangsläufig gekoppelt und dementsprechend miteinander in Wirkverbindung stehen. Die durch das Formschlusselement und das Formschlussgegenelement bewirkte Führung zwischen dem Antriebsgehäuse und dem Pumpenmodul verhindert dabei Fehlbedienungen.

Das Formschlusselement und das Formschlussgegenelement bilden vorzugsweise einen Bajonettverschluss aus, der dem Pumpengehäuse relativ zu dem Antriebsgehäuse zunächst eine axiale Bewegung aufprägt. Diese axiale Bewegung ist eine axiale Bewegung relativ zu einer im Wesentlichen zylindrischen Ausnehmung. Am Ende dieser axialen Bewegung wird durch Zusammenwirken von Formschlusselement und Formschlussgegenelement eine schwenkende Relativbewegung aufgeprägt, die sich im Wesentlichen rechtwinklig zu der axialen Bewegung erstreckt. Durch den zweiten Teil der durch den Bajonettverschluss vorgegebenen Schwenkbewegung wird auch sicher gestellt, dass die Verbindung zwischen dem Pumpenmodul und dem Antriebsgehäuse gesichert ist. So können bei Bajonettverschlüssen übliche Rastmittel oder Vorsprünge vorgesehen sein, die ein versehentliches Zurückdrehen des Pumpenmoduls relativ zu dem Antriebsgehäuse verhindern. Bei der hier diskutierten Ausgestaltung kann die Ausnehmung durch das Pumpengehäuse vorgegeben sein, in welche ein Vorsprung des Antriebsgehäuses eingreift. Mit Blick auf eine kompakte Ausgestaltung ist es indes zu bevorzugen, die Ausnehmung an dem Antriebsgehäuse vorzusehen. Dabei liegt das Antriebselement vorzugsweise in der Ausnehmung frei, ist indes bevorzugt innerhalb der Ausnehmung aufgenommen. Dementsprechend überragt das Antriebselement üblicherweise nicht die Ausnehmung in Längsrichtung, die üblicherweise Bewegungsrichtung des Antriebselementes ist, nach außen. Bei dieser Ausgestaltung ist das Pumpenmodul so ausgebildet, dass das Pumpengehäuse zumindest teilweise in die Ausnehmung einsetzbar ist.

Gemäß einer bevorzugten Weiterbildung der vorliegenden Erfindung hat das Pumpengehäuse eine Ausnehmung, in der das Antriebsgegenelement frei liegt. Zum Einbringen in eine an dem Antriebsgehäuse ausgebildete Ausnehmung weist das Pumpengehäuse bevorzugt einen Hülsenabschnitt auf, welcher das Antriebsgegenelement umgibt und das Formschlussgegenelement ausformt. Auch bei dieser Ausgestaltung befindet sich ein Antriebsgegenelement zumindest überwiegend mit seinem nach außen freiliegenden Anschlussende innerhalb der Ausnehmung, die im Folgenden als Pumpenausnehmung bezeichnet werden soll, sodass dieses Anschlussende des Antriebsgegenelements geschützt zwar innerhalb des Pumpengehäuses aufgenommen ist, jedoch auch innerhalb des Pumpengehäuses freiliegt.

Gemäß einer bevorzugten Weiterbildung der vorliegenden Erfindung ist/sind die Ausnehmung und/oder das Pumpengehäuse zumindest teilweise rotationssymmetrisch ausgestaltet. Die rotationssymmetrische Ausbildung betrifft dabei insbesondere eine Hüllfläche beispielsweise an innen umfängliche Flächenabschnitte der Ausnehmung und/oder außenumfängliche Abschnitte insbesondere des Hülsenabschnitts des Pumpengehäuses. Die Formschlusselemente bzw. Formschlussgegenelemente liegen regelmäßig innerhalb bzw. außerhalb dieser Hüllfläche. Die rotationssymmetrische Ausgestaltung ergibt Führungsflächen, die beim Fügen von Pumpenmodul und Antriebsgehäuse zumindest die axiale Bewegung führen. Diese axiale Bewegung ist üblicherweise eine Bewegung die parallel zu der Mittellängsachse der Ausnehmung bzw. eines im Wesentlichen zylindrischen Hülsenabschnitts ausgerichtet ist. Während die rotationssymmetrische Hüllfläche die axiale Bewegung führt, bewirkt das Zusammenwirken von Formschlusselement und Formschlussgegenelement zwar auch eine axiale Führung, insbesondere aber die Zwangsführung der Schwenkbewegung bis zum Erreichen der Endposition.

Gemäß einer bevorzugten Weiterbildung der vorliegenden Erfindung bilden das Formschlusselement und das Formschlussgegenelement eine Führung aus, die eine eindeutige Ausrichtung des Pumpengehäuses relativ zu dem Antriebsgehäuse beim Fügen vorgibt. Dabei ist insbesondere an eine Ausgestaltung gedacht, die eine Poka-Yoke-Funktion erfüllt und somit vorgibt, dass das Pumpengehäuse nur in einer einzigen, ggf. zwei jeweils um 180° verdrehten Positionen relativ zu dem Antriebsgehäuse durch Zusammenwirken von Formschluss- und Formschlussgegenelement gefügt werden kann. So können auf dem Umfang der Ausnehmung und/oder eines Hülsenabschnitts mehrerer Formschlusselemente und Formschlussgegenelemente vorgesehen sein, die jeweils für sich einander genau zugeordnet sind, sodass die jeweils zugehörigen Paare von Formschlusselement und Formschlussgegenelement notwendiger Weise zur Überdeckung gebracht werden müssen, um ein Fügen des Pumpenmoduls an dem Antriebsgehäuse zu ermöglichen. Auch diese Ausgestaltung verhindert sicher ein fehlerhaftes Fügen von Pumpengehäuse und Antriebsgehäuse und damit ein unzureichendes mechanisches Koppeln von Antriebselement und Antriebsgegenelement.

Gemäß einer bevorzugten Weiterbildung der vorliegenden Erfindung sind das Formschlusselement und das Formschlussgegenelement so ausgebildet, dass das Pumpengehäuse beim Fügen und am Ende der axialen Bewegung relativ zu dem Antriebsgehäuse um einen Winkel von zwischen 10° und 90°, bevorzugt um einen Winkel von 25-35°, also 30° +/- 5° schwenkbar ist. Eine solche Schwenkbarkeit kann üblicherweise ohne Umgreifen der das Pumpengehäuse haltenden Hand durch den Benutzer erreicht und somit komfortabel verwirklicht werden. Damit lässt sich das Fügen einhändig und komfortabel durchführen.

Gemäß einer bevorzugten Weiterbildung der vorliegenden Erfindung ist das Antriebselement als Antriebsstößel ausgebildet. Das Antriebsgegenelement wird bevorzugt einteilig als Teil eines Pumpkolbens ausgebildet. Mit anderen Worten wird das Antriebsgegenelement durch den Pumpkolben ausgeformt. Es versteht sich von selbst, dass der zum Pumpen verwirklichte Teil des Kolbens in einem Zylinder üblicherweise innerhalb des Pumpengehäuses aufgenommen ist, wohingegen das antriebsseitige andere freie Ende des Pumpkolbens das Antriebsgegenelement ausformt. Die durch das Antriebselement und dem Pumpkolben ausgeformte Führung zwischen dem Pumpenmodul und dem Antriebsgehäuse in axialer Richtung ist dabei so ausgebildet, dass das Antriebselement an eine Anschlagfläche des Pumpkolbens anstößt, bevor die Endposition erreicht ist. Durch diese Ausgestaltung wird erreicht, dass das Antriebselement und das Antriebsgegenelement in axialer Richtung nach dem Fügen von Pumpengehäuse und Antriebsgehäuse gegeneinander anliegen. Dabei stößt der Antriebsstößel mit seiner Anschlagfläche bevorzugt gegen die Gegenfläche des Pumpkolbens an, bevor das Fügen von Antriebsgehäuse und Pumpmodul in axialer Richtung beendet ist. Mit anderen Worten trifft die Anschlagfläche beim axialen Fügen die Gegenfläche. Antriebselement und Antriebsgegenelement sind damit zumindest stirnseitig gegeneinander angelegt. Diese Anlage wird zumindest bei Erreichen der Endposition erreicht. Vorzugsweise kann diese Anlage auch vor Erreichen der Endposition bewirkt werden. Die verbleibende axiale Verschiebebewegung zwischen dem Pumpenmodul und dem Antriebsgehäuse wird dann durch eine axiale Relativbewegung von Pumpkolben und/oder Antriebsstößel kompensiert, sodass sich die relative Lage von Antriebselement und Antriebsgegenelement trotz einer axial Fügebewegung nicht verändert. Hierdurch wird die Sicherheit erhöht, so dass am Ende der axialen Fügebewegung das Antriebselement sicher endseitig gegen den Pumpkolben angelegt ist.

Üblicherweise wird zur Vereinfachung der Ausgestaltung der antriebsseitige Antriebskolben axial unverschieblich gehalten, wohingegen das dem Pumpmodul zugeordnete Antriebsgegenelement axial verschieblich montiert ist.

Mit Blick auf die mit der Erfindung gelehrte zwangsläufige formschlüssige Verbindung infolge einer Schwenkbewegung zwischen dem Pumpengehäuse und dem Antriebsgehäuse wird gemäß einer bevorzugten Weiterbildung der vorliegenden Erfindung vorgeschlagen, dass das Antriebselement oder das Antriebsgegenelement einen Hammerkopf aufweist und der jeweils andere von den beiden Elementen eine zum Übergreifen des Hammerkopfes angepasst ausgebildete Hammerkopfaufnahme aufweist. Die Hammerkopfaufnahme ist dabei so auf die Ausgestaltung des Hammerkopfes abgestimmt, dass die Schwenkbewegung am Ende der Fügebewegung, d.h. nach Beendigung der axialen Relativbewegung und im Rahmen der Schwenkbewegung dazu führt, dass der Hammerkopf formschlüssig von der Hammerkopfaufnahme übergriffen und damit beide Elemente des Antriebs in axialer Richtung beidseitig formschlüssig miteinander verbunden sind. So führt eine alternierende axiale Bewegung das Antriebselementes zwangsläufig auch zu einer entsprechenden alternierenden Bewegung des Antriebsgegenelementes, nachdem das Pumpenmodul die Endposition erreicht hat und der Antrieb eingeschaltet worden ist.

Mit Blick auf eine exakte axiale Einkopplung durch formschlüssige Verbindung von Antriebelement und Antriebsgegenelement wird gemäß einer bevorzugten Weiterbildung der vorliegenden Erfindung vorgeschlagen, das die Hammerkopfaufnahme ausbildende Element verdrehfest vorzusehen. Dabei kann das den Hammerkopf ausbildende Element drehbar gelagert sein, und zwar insbesondere dann, wenn der Hammerkopf selbst rotationssymmetrisch ausgebildet ist, sodass dessen Ausrichtung relativ zu der Hammerkopfaufnahme die Qualität der herzustellenden formschlüssigen Verbindung im Rahmen des Fügens nicht beeinflusst.

Zur weiteren Erhöhung der Sicherheit gegen unrichtiges Fügen wird gemäß einer weiteren bevorzugten Ausgestaltung der vorliegenden Erfindung vorgeschlagen, die Hammerkopfaufnahme mit einer den Hammerkopf übergreifenden Klaue zu versehen. Der Übergriff des Hammerkopfes durch die Klaue bewirkt dabei die axiale Festlegung von Antriebselement und Antriebsgegenelement. Als Festlegung in diesem Sinne ist allerdings auch eine Ausgestaltung zu verstehen, bei der eine zyklische axiale Bewegung des Antriebselementes mitunter nicht vollständig auf das Antriebsgegenelement übertragen wird, da beide ein gewisses axiales Spiel zulassen. Ein solches Spiel, welches zum Schlagen der beiden Elemente führt, was insbesondere bei hochfrequentem Antrieb zu unerwünschten Geräuschen und Verschleiß führen kann, sollte indes vermieden werden. So ist die Hammerkopfaufnahme üblicherweise so ausgebildet, dass diese den Hammerkopf im Wesentlichen ohne axiales Spiel in sich aufnimmt. Die nach der hier diskutierten bevorzugten Weiterbildung vorgesehene Klaue bildet indes nach der Weiterbildung auch einen Anschlag aus. Dieser Anschlag ist wirksam vor Erreichen der Endposition und wirkt mit dem Hammerkopf zusammen, sofern sich dieser auf gleicher Höhe wie die Klaue befindet und zwar derart, dass ein Schwenken in die Endposition verhindert wird. Mit anderen Worten verhindert das Zusammenwirkung von Hammerkopf und Anschlag das Erreichen der Endposition. Der Anschlag ist in einer Schwenkbewegung des Pumpenmoduls relativ zu dem Antriebsgehäuse wirksam, er wirkt danach radial.

Als für sich erfindungswesentlich wird eine Antriebseinrichtung der zuvor diskutierten Vorrichtung zur Erzeugung eines Flüssigkeitsstrahls angesehen und in einem nebengeordneten Anspruch unter Schutz gestellt. Danach hat die Antriebseinheit einen Antrieb, der in einem Antriebsgehäuse vorgesehen ist. Das Antriebsgehäuse weist zumindest ein Formschlusselement auf, mit welchem das zuvor erwähnte Pumpenmodul an dem Antriebsgehäuse festlegbar ist. Die Festlegung erfolgt dabei formschlüssig, bevorzugt in der zuvor beschriebenen Art mittels Bajonettverschluss. Das Antriebsgehäuse ist ferner mit einer Ausnehmung versehen. In dieser Ausnehmung liegt ein Antriebselement des Antriebs frei. Das Antriebselement ist exzentrisch zu einer Mittellängsachse der Ausnehmung angeordnet. Da die Ausnehmung des Antriebsgehäuses ein Schwenken des zumindest teilweise in die Ausnehmung eingesetzten Pumpenmoduls erlauben soll, ist die Ausnehmung üblicherweise mit einer rotationssymmetrischen Innenumfangsfläche ausgebildet. Entsprechendes gilt für den in die Ausnehmung eingebrachten Teilbereich des Pumpenmoduls. Dieser Teilbereich ist üblicherweise als zylindrischer Abschnitt mit kreisförmiger Außenumfangsfläche ausgebildet. Aufgrund dieser Ausgestaltung ist es möglich, das Pumpmodul jedenfalls teilweise in die Ausnehmung des Antriebsgehäuses einzusetzen und durch Schwenken formschlüssig zu verbinden.

Das Antriebselement weist ein Formschlussmittel zur formschlüssigen Verbindung des Antriebselementes mit einem Antriebsgegenelement des Pumpenmoduls durch Schwenken des Pumpenmoduls um die Mittellängsachse auf. Das Formschlussmittel ist so ausgebildet, dass beim Schwenken des Pumpenmoduls um die Mittellängsachse der Ausnehmung zur formschlüssigen Festlegung des Pumpenmoduls an dem Antriebsgehäuse mittels des Formschlusselementes das Antriebselement der Antriebseinheit mit einem Antriebsgegenelement des Pumpenmoduls verbunden wird. Bei dem erfindungsgemäßen Pumpenmodul sind dementsprechend vorzugsweise das Formschlussgegenelement zur Festlegung des Pumpenmoduls an dem Antriebsgehäuse und das Formschlussmittel zum Verbinden von Antriebselement und Antriebsgegenelement jeweils exzentrisch zu der Mittellängsachse der Ausnehmung angeordnet. So bietet die erfindungsgemäße Antriebseinheit die Möglichkeit, sowohl das Pumpenmodul als Ganzes als auch das Antriebselement der Antriebseinheit mit dem zugehörigen Antriebsgegenelement des Pumpenmoduls im Rahmen einer einheitlichen Schwenkbewegung zwangsläufig zu koppeln.

Als für sich erfindungswesentlich wird auch das in einem weiteren nebengeordneten Anspruch spezifizierte Pumpenmodul der zuvor diskutierten Vorrichtung zur Erzeugung eines Flüssigkeitsstrahls angesehen. Dieses Pumpenmodul hat ein Pumpengehäuse mit zumindest einem Formschlussgegenelement, mit welchem das Pumpenmodul an einem Antriebsgehäuse eines Antriebs festlegbar ist. Das Pumpengehäuse ist ferner zum Einbringen in eine Ausnehmung des Antriebsgehäuses angepasst ausgebildet und hat hierzu einen entsprechenden zylindrischen Abschnitt. Der zylindrische Abschnitt ist üblicherweise mit rotationssymmetrischer Außenumfangsfläche ausgebildet und nach Art einer Passung an die Abmessung der Ausnehmung angepasst. Das Pumpenmodul hat desweiteren ein Antriebsgegenelement, welches exzentrisch zu einer Mittellängsachse des zylindrischen Abschnitts angeordnet ist. Das Antriebsgegenelement ist mit einem Formschlussgegenmittel zur formschlüssigen Koppelung des Antriebsgegenelementes mit einem in der Ausnehmung freiliegenden Antriebselement des Antriebs durch Schwenken des Pumpenmoduls um die Mittellängsachse des zylindrischen Abschnitts versehen. Diese Schwenkbewegung erfolgt beim formschlüssigen Festlegen des Pumpenmoduls an dem Antriebsgehäuse über das Formschlusselement des Pumpengehäuses.

Weitere Einzelheiten und Vorteile der vorliegenden Erfindung entnehmen Sie der nachfolgenden Beschreibung eines Ausführungsbeispiels in Verbindung mit der Zeichnung. In dieser zeigen:
- Figur 1: Eine perspektivische Ansicht eines Ausführungsbeispiels einer erfindungsgemäßen Vorrichtung zur Erzeugung eines Fluidstrahles;
- Figur 2: das Detail nach Figur 1 in vergrößerter Darstellung ohne Pumpenmodul;
- Figur 3: das Detail nach Figur 2 in einer Draufsicht;
- Figur 4: eine perspektivische Draufsicht auf einen Endbereich des in Figur 1 gezeigten Pumpenmoduls;
- Figur 5: eine stirnseitige Draufsicht auf den in Figur 4 gezeigten Endbereich;
- die Figuren 6a-c: eine Abfolge von Schritten beim Fügen des Pumpenmoduls;
- Figur 7: eine perspektivische, teilweise transparente Seitenansicht des Pumpenmoduls des Ausführungsbeispiels;
- Figur 8a-d: , halb geschnittene Ansichten von Antriebselement und Antriebsgegenelement des Ausführungsbeispieles und deren Position relativ zueinander beim Fügen;
- Figur 9a-c: teilweise geschnittene Draufsichten auf die zusammenwirkenden Enden von Antriebselement und Antriebsgegenelement und deren relative Lage beim Schwenken im Rahmen des Fügens; und
- Figur 10: eine perspektivische, teilweise transparente Seitenansicht.

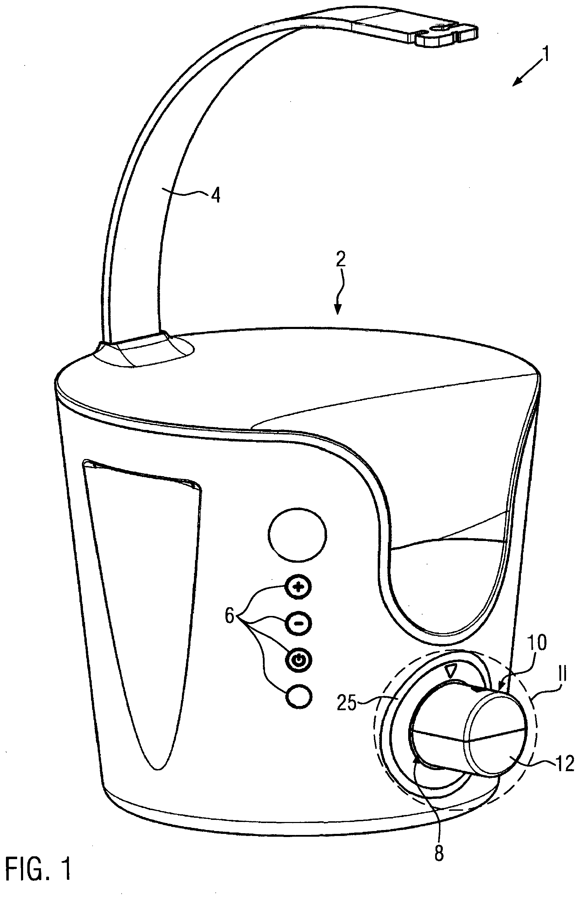

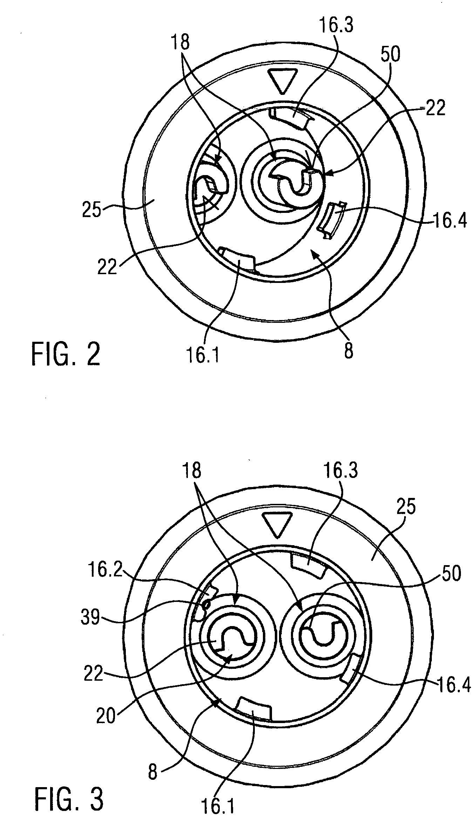

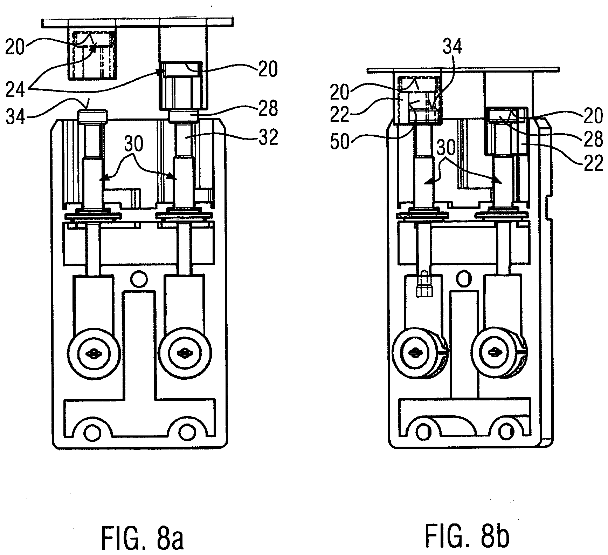

Die Figur 1 zeigt eine perspektivische Seitenansicht eines Ausführungsbeispiels eine Antriebseinheit 1 mit einem in einem Antriebsgehäuse 2 vorgesehenen Antrieb, bei dem es sich um einen elektrischen Antrieb handelt. Von dem Antriebsgehäuse 2 ragt eine Halterung 4 zur Halterung eines Flüssigkeitsbeutels ab. An dem Antriebsgehäuse 2 liegen ferner verschiedene Bedienelemente 6 frei, die der Steuerung des Antriebs sowie dem Ein- bzw. Ausschalten des Antriebs dienen. Bezugszeichen 8 kennzeichnet eine im Wesentlichen zylindrische Ausnehmung, in die ein mit Bezugszeichen 10 gekennzeichnetes Pumpenmodul eingesetzt ist, welches die Ausnehmung 8 überragt. Das Pumpenmodul 10 hat ein aus zwei Gehäuseschalen gebildetes Pumpengehäuse 12.Wie die Figuren 2 und 3 verdeutlichen, hat das Antriebsgehäuse 2 nach innen in die Ausnehmung 8 vorspringende Nocken 16, die Ausführungsbeispiele für Formschlusselemente der vorliegenden Erfindung sind. Es sind vorliegend vier Nocken 16 auf dem Umfang verteilt vorgesehen. Die mit Bezugszeichen 16.4 gekennzeichnete Nocke hat eine geringere radiale Erstreckung und eine geringere Erstreckung in Umfangsrichtung als die übrigen Nocken 16.1 bis 16.3. In der Ausnehmung 8 liegen ferner Antriebselemente in Form von Antriebsstößeln 18 frei, die mit dem innerhalb des Antriebsgehäuses 2 vorgesehenen Antrieb verbunden und in Längsrichtung zyklisch angetrieben hin und her beweglich sind. Die Antriebsstößel 18 bilden eine Anschlagfläche 20 aus. Vorliegend sind zwei Antriebsstößel 18 vorgesehen. Die Anschlagfläche 20 wird durch eine in der Draufsicht C-förmige Klaue 22 überragt, die zwischen sich und der Anschlagfläche 20 eine Hammerkopfaufnahme 24 ausbildet. Die entsprechenden Details sind insbesondere in Figur 8a deutlich gekennzeichnet.

In den Figuren 1 und 2 ist mit Bezugszeichen 25 eine Dichtung aus einem gummielastischen Material gekennzeichnet, die sich zwischen der Außenseite des Antriebsgehäuses 2 und einer Mündung zu der Ausnehmung erstreckt. Die Ausnehmung 8 wird dabei von einem dem Antrieb zugeordneten Topf gebildet, der die beiden Antriebsstößel 18 umschließt. Dieser Topf ist gewissen Vibrationen ausgesetzt, die durch den Antrieb begründet sind. Die Dichtung 25 verhindert eine unmittelbare Übermittlung dieser Schwingungen an das Antriebsgehäuse 2.

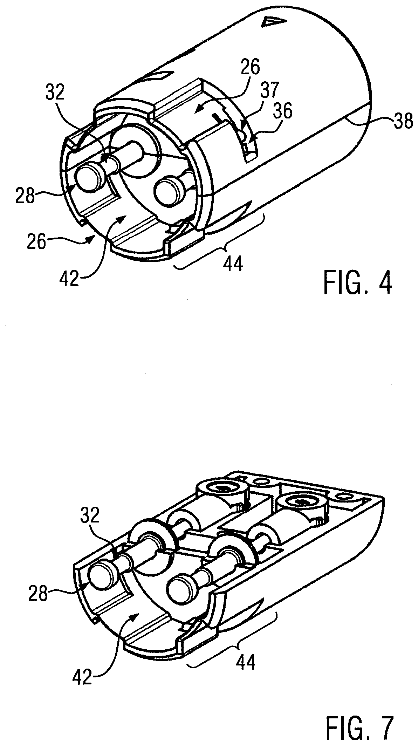

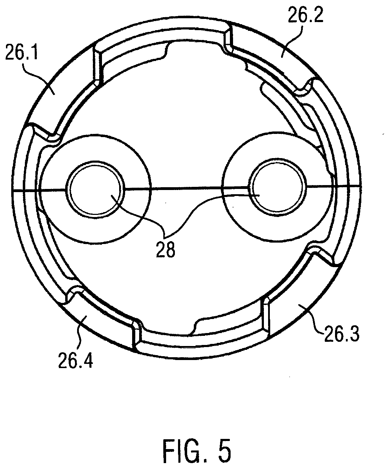

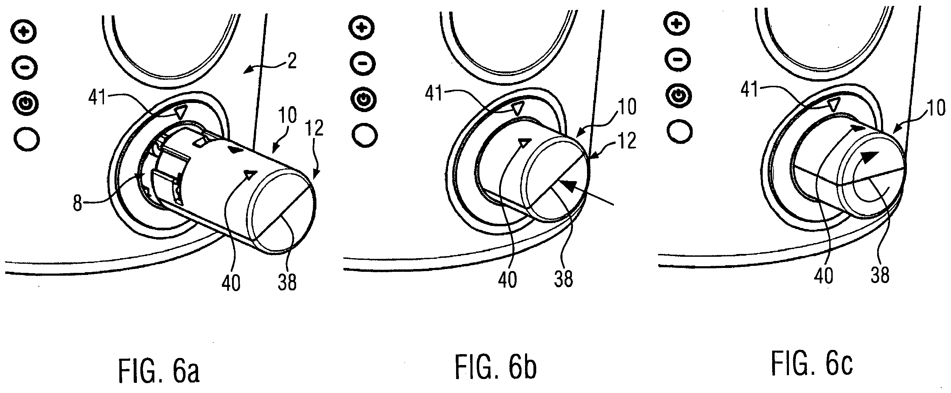

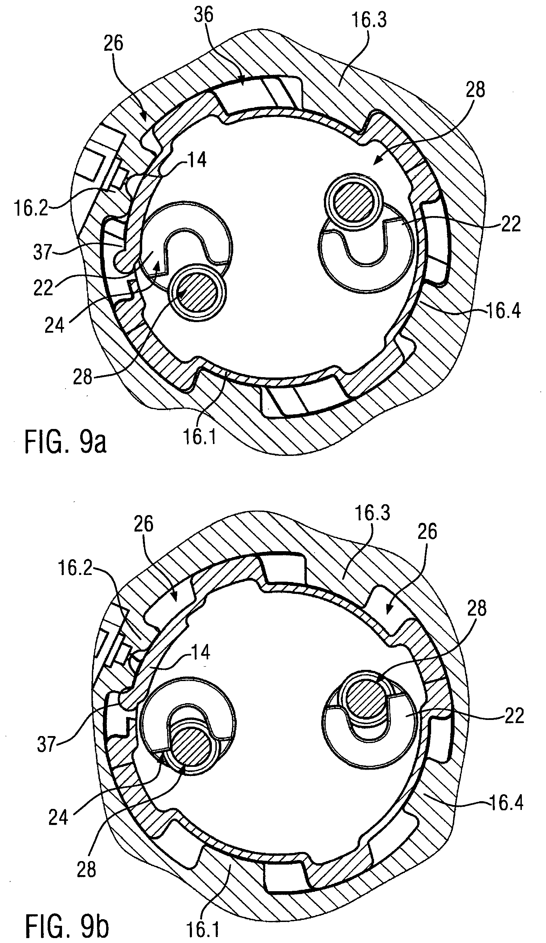

Wie insbesondere die Figuren 4 und 5 erkennen lassen, hat das Pumpenmodul 10 mit Bezugszeichen 26 gekennzeichnete, sich in axialer Richtung in Bezug auf das zylindrische Pumpengehäuse 12 erstreckende Nuten 26, die Formschlussgegenelemente im Sinne der vorliegenden Erfindung beispielhaft ausbilden. Diese Nuten 26 sind am Außenumfang des Pumpengehäuses 12 sich streng in axialer Richtung erstreckend vorgesehen. Die mit Bezugszeichen 26.4 gekennzeichnete Nut hat eine geringere radiale Tiefe und eine geringere Breite in Umfangsrichtung und ist zur exakten Aufnahme der kleineren Nocke 16.4 angepasst ausgebildet. Durch das Zusammenwirken insbesondere der kleineren Nocke 16.4 mit der kleineren Nut 26.4 wird eine eineindeutige Ausrichtung des Pumpengehäuses 12 beim Fügen, d.h. beim Einschieben des Pumpengehäuses 12 in die Ausnehmung 8 vorgegeben. Damit kann das Pumpengehäuse 12 nur in einem Winkel rechtwinklig zu einer in Figur 6c gezeigten Endlage um 30° versetzt eingeführt werden. Diese geschwenkte Lage ist in Figur 6b und 9a verdeutlicht. Dabei verdeutlichen die Bezugszeichen 28 in Figur 9a jeweils zwei Hammerköpfe, die an freien Enden jeweils eines Pumpkolbens 30 vorgesehen sind, die jeweils ein Ausführungsbeispiel eines Antriebsgegenelementes im Sinne der vorliegenden Erfindung ausbilden. Der Hammerkopf 28 überragt einen endseitigen Zylinderabschnitt 32 jedes einzelnen Pumpkolbens 30, der einen geringeren Durchmesser als der übrige Pumpkolben 30 hat. Der Hammerkopf 28 definiert das stirnseitige, anschlussseitige Ende des Pumpkolbens 30 und bildet hier eine Gegenfläche 34 zu der Anschlagfläche 20 aus.

Wie insbesondere Figur 4 verdeutlicht, geht von einem inwärtigen Ende der Nut 26 eine Quernut 36 ab. Die Nut 26 bildet dementsprechend zusammen mit der Quernut 36 eine Führung eines Bajonettverschlusses mit dem jeweiligen Nocken 16, um zunächst eine axiale Einführbewegung zu führen, die ihr Ende dann findet, wenn die Nocken 16 gegen das innenseitige untere Ende der Nuten 26 anstoßen, um danach in einer Schwenkbewegung in die Quernut 36 schwenkt und damit axial arretiert zu werden. In der endseitig gegen die Quernut 36 anliegenden Endlage kann ein Rastvorsprung wirksam sein, der eine Verdrehsicherung zwischen dem Pumpenmodul 10 und dem Antriebsgehäuse 2 ausbildet, sodass das Pumpenmodul 10 in seiner Endlage gesichert ist.

Die in Fig. 3 gezeigten Nocken 16 sind nicht identisch ausgebildet. Identisch sind vielmehr lediglich die Nocken 16.1 und 16.2, zu denen die zugeordneten Nuten entsprechend angepasst ausgeformt sind. Die Nocke 16.4 ist in ihrer umfänglichen Erstreckung weniger breit als die Nocken 16.1 bis 16.3. Ihr ist eine entsprechend schmale Nut 26 zugeordnet. Die entsprechende Nut 26 passt dementsprechend lediglich mit der Nocke 16.4 zusammen, wodurch eine eindeutige Ausrichtung zwischen dem Pumpenmodul 10 und der Ausnehmung 8 vorgegeben ist.

In Fig. 4 ist ferner innerhalb der Quernut 36 ein Rast-und Schaltvorsprung 37 eingezeichnet, der in der Quernut 36 freiliegt und fest an dem Pumpengehäuse 12 ausgebildet ist. Diesem Rast- und Schaltvorsprung 37 ist ein mittig in der Nocke 16.2 vorgesehener Schalter 39 zugeordnet. Der Schalter 39 ist in radialer Richtung nach innen in Bezug auf die Ausnehmung 8 vorgespannt und wirkt dementsprechend mit dem Rast- und Schaltvorsprung 37 zusammen. Durch Betätigung dieses Schalters durch den Rast- und Schaltvorsprung 37 wird erst die Möglichkeit gegeben, die Antriebsstößel 18 anzutreiben. Ist dementsprechend das Pumpenmodul 10 nicht in der vorgeschriebenen Weise mit der Antriebseinheit 1 verbunden, kann die Antriebseinheit nicht betrieben werden. Zusätzlich sind das Antriebsgehäuse 2 und das Pumpengehäuse 12 mit einander zugeordneten, vorzugsweise elektronischen Schlüssel-Schloss-Mitteln versehen. So kann beispielsweise an dem Pumpengehäuse 12 ein RFID-Tag vorgesehen sein, welches durch eine Leseeinheit, die an dem Antriebsgehäuse 2 vorgesehen ist, erkannt wird. Erst bei Anwesenheit eines entsprechenden RFID-Tags wird von einer in dem Antriebsgehäuse 2 vorgesehenen Logikeinheit der Antriebsstößel 18 freigegeben.

Die Figuren 6a bis c zeigen das Einführen des Pumpenmoduls 10 in die Ausnehmung 8. Wie bereits zuvor erwähnt, wird das Pumpenmodul 10 um 30° im Gegenuhrzeigersinn relativ zu der Endlage zunächst geschwenkt, um die Nocken 16 mit den Nuten 26 zur Überdeckung zu bringen (vgl. Figur 6a). Die geschwenkte Position wird durch eine Teilungsebene visualisiert, die zwischen zwei das Pumpengehäuse 12 bildenden Gehäuseelementen vorgesehen ist. In dieser relativen Ausrichtung kann das Pumpenmodul 10 nunmehr in die Ausnehmung 8 eingeschoben werden. Diese axiale Einschiebebewegung wird geführt durch die Nocken 16, die in die korrespondierend hierzu ausgebildeten Nuten 26 eingreifen. In der Darstellung nach Figur 6b ist dieses axiale Einschieben, das in Figur 6b mit einem gradlinigen Pfeil verdeutlicht wird, des Pumpengehäuses 12 beendet. Das Pumpengehäuse 12 ist nunmehr maximal in die Ausnehmung 8 eingeschoben. Danach wird das Pumpengehäuse um 30° im Uhrzeigersinn geschwenkt, wie dies der Pfeil in Figur 6c andeutet. Nach dieser Schwenkbewegung um 30° hat das Pumpenmodul 10 seine Endposition erreicht. Die Endposition wird dem Benutzer auch durch einen auf dem Außenumfang des Pumpengehäuses 2 vorgesehenen Richtungspfeil 40 angeben, der in der Endposition mit einer Gegenmarkierung 41, die an dem Antriebsgehäuse 2 versehen ist, fluchtet. Der Richtungspfeil gibt auch die Richtung des Einführens für das Pumpengehäuse 2 in die Ausnehmung 8 vor.

Wie die zuvor diskutierten Figuren verdeutlichen, liegen die Antriebsstößel 18 innerhalb der Ausnehmung 8 frei, sind allerdings von dieser umfänglich überdeckt und damit geschützt eingehaust. Lediglich die zylindrische Ausnehmung 8 gibt einen Zugang zu den Antriebsstößeln 18 frei.

In ähnlicher Weise weist das Pumpengehäuse 2 eine Pumpenausnehmung 42 auf, die von einem mit Bezugszeichen 44 gekennzeichneten Hülsenabschnitt 44 des Pumpengehäuses 12 umgeben ist, welcher an seiner Außenumfangsfläche die Nuten 26, 36 ausformt. Die Pumpenkolben ragen mit ihrem Anschlussende geringfügig über den Hülsenabschnitt 44 axial hinaus, sind aber ansonsten innerhalb des Hülsenabschnitts 44 aufgenommen und lediglich axial durch die Öffnung der Pumpenausnehmung 42 zugänglich.

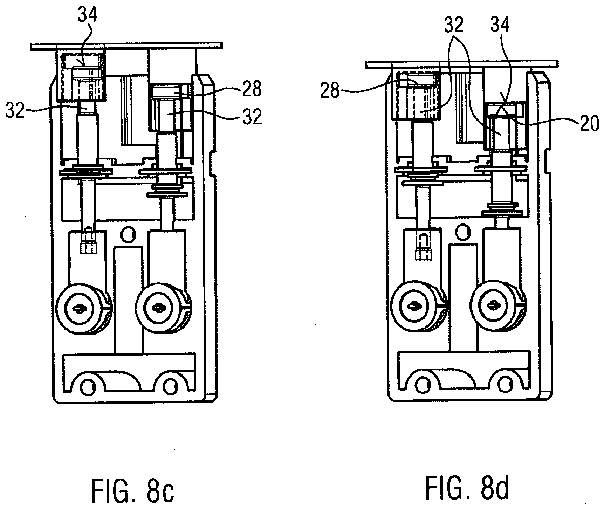

Beim Fügen von Pumpengehäuse 12 und Antriebsgehäuse 2 werden die Antriebsstößel 18 und die Pumpkolben 30 einander angenähert, wie dies die Figuren 8a und 8b verdeutlichen. Aufgrund der axialen Führung der Nocken 16 in den Nuten 26 befindet sich die durch den Hammerkopf 28 gebildete Gegenfläche 34 zumindest teilweise über der durch den Antriebsstößel 18 gebildeten Anschlagfläche (vgl. Figur 9a). So führt eine fortschreitende axiale Bewegung schließlich dazu, dass der Pumpkolben 30 endseitig gegen die Anschlagfläche 20 angelegt wird, und zwar auch dann, wenn die alternierend axial bewegten Antriebsstößel 18 unterschiedlich weit in die Ausnehmung 8 hineinragen, wie dies bei der Darstellung nach den Figuren 8a bis d unterstellt ist. Mit zunehmender Annäherung des Pumpengehäuses 12 an das Antriebsgehäuse 2 ergibt sich danach keine weitere relative axiale Bewegung zwischen dem in den Figuren 8a bis d oben gezeigten Paar von Antriebselement 18 und Pumpkolben 30. Nach einer weiteren in den Figuren 8b, 8c und 8d verdeutlichten axialen Verschiebebewegung des Pumpengehäuses 12 relativ zu dem Antriebsgehäuse 2 stößt schließlich auch der untere Pumpkolben 30 mit seiner Gegenfläche 34 gegen die Anschlagfläche 20 des zugeordneten Antriebsstößels 18.

Der jeweilige Hammerkopf 28 der beiden Pumpkolben 30 befindet sich dabei in einer exzentrischen Position relativ zu dem Mittelpunkt des Antriebsstößels 18, die in Figur 9a gezeigt ist. Üblicherweise wird nach dem axialen Anlegen beider Pumpkolben 30 an die Antriebsstößel 18 das Pumpengehäuse 12 um einen weiteren, geringfügigen Weg axial relativ zu dem Antriebsgehäuse 2 verschoben, sodass sichergestellt wird, dass bis zum Erreichen der axialen Endlage beim Fügen von Pumpmodul 10 und Antriebsgehäuse 2 jederzeit zuverlässig eine axiale Anlage des Pumpenkolbens 30 an dem Antriebsstößel 18 erreicht wird, bevor das Pumpenmodul 10 relativ zu dem Pumpengehäuse 12 geschwenkt wird. Jedenfalls sollte die Ausgestaltung so sein, dass bei jeder erdenklichen Positionierung des Antriebsstößels 18, selbst bei einer Positionierung des Antriebsstößels 18 in der tiefsten Lage innerhalb der Ausnehmung 8 eine sichere Anlage des Pumpkolbens 30 gegen den Antriebsstößel 18 nach Beendigung der axialen Einschiebebewegung erreicht ist.

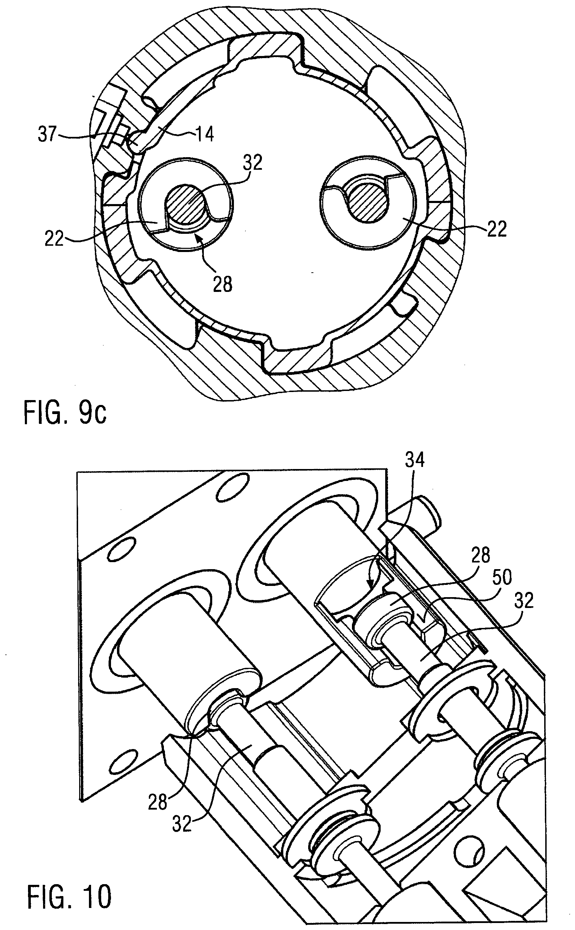

Nach Erreichen dieser axialen Endlage wird das Pumpenmodul 10 nunmehr im Uhrzeigersinn geschwenkt. Dadurch werden - wie die Figuren 9a bis 9c verdeutlichen - die exzentrisch zu dem Mittelpunkt dieser Schwenkbewegung angeordneten Hammerköpfe 28 mit ihrer Gegenfläche 34 gleitend auf der Anschlagfläche 20 relativ zu dem Antriebsstößel 18 verschoben, und zwar in einer Ebene, die sich rechtwinklig zu der Einschieberichtung erstreckt. Die zuvor exzentrische Anordnung der Pumpkolben 30 relativ zu den Antriebsstößeln 18 gemäß Figur 9a nähert sich danach über eine in Figur 9b gezeigte Zwischenposition der in Figur 9c gezeigten Endposition an. In dieser Endposition stoßen die Nocken 16 gegen Anschläge an, die durch die Quernuten 36 gebildet sind. Das Pumpengehäuse 12 wird üblicherweise gegen das Antriebsgehäuse 2 verriegelt. Die Pumpkolben 30 sind im Wesentlichen konzentrisch zu den Antriebsstößeln 18 angeordnet. Jede Klaue 22 übergreift den zugeordneten Hammerkopf 28. So ist der Hammerkopf 28 durch Übergriff der die Klaue 26 aufweisenden Hammerkopfaufnahme 24 axial formschlüssig gehalten. Üblicherweise ist die Hammerkopfaufnahme 24 in axialer Richtung exakt auf die Höhe des Hammerkopfes 28 abgestimmt, sodass sich eine spielfreie axiale formschlüssige Verbindung zwischen dem Antriebsstößel 18 und dem Pumpkolben 30 ergibt. Wie die Fign. 9a-c erkennen lassen, befindet sich der Rast- und Schaltvorsprung 37 an einem freien Ende eines durch das Pumpengehäuse 12 ausgebildeten Federarms 14, der in eine durch die Nocke 16.2 ausgeformte Rastmulde einrastet (vgl. Fig. 9c).

Das Lösen des Pumpenmoduls 10 erfolgt in umgekehrter Richtung. Auch hierbei werden die jeweiligen Hammerköpfe 28 durch das Schwenken zunächst außer Eingriff der Hammerkopfaufnahmen 24 gebracht. Sodann kann das Pumpenmodul 10 aus der Ausnehmung 18 herausgezogen werden. Diese Bewegung wird geführt durch das Zusammenwirken der Nocken 16 mit den axialen Nuten 26.

Die Figur 10 verdeutlicht eine Sicherung des gezeigten Ausführungsbeispiels, die verhindert, dass bei unzureichendem Übergriff der Hammerköpfe 28 die Schwenkbewegung in die Endposition vollzogen werden kann. Denn die Klaue 22 bildet mit ihrer Außenkontur einen Anschlag 50 aus, der radial wirksam ist, d.h. beim Schwenken des Pumpengehäuses 12 mit dem Hammerkopf 28 zusammenwirkt, wie dies in Figur 10 gezeigt ist, und dementsprechend eine Schwenkbewegung gemäß den Figuren 9a bis 9c verhindert. Eine solche Schwenkbewegung kann theoretisch möglich sein, wenn die Nocken 16 das untere Ende der Längsnuten 26 erreicht haben, d.h. das Pumpengehäuse 12 maximal in die Ausnehmung 8 eingeschoben ist, ein Pumpkolben 30 beispielsweise aufgrund einer manuellen Verschiebung des Pumpkolbens 30 aber nicht in der nach vorne in Richtung auf das Antriebsgehäuse 2 vorspringenden Position gehalten ist. Durch diesen radialen Anschlag 50 wird dementsprechend eine unrichtige Montage des Pumpenmodules 10 an dem Antriebsgehäuse 2 vermieden. Dieser Anschlag 50 ist so lange wirksam, wie sich der Hammerkopf 28 in axialer Richtung, d.h. Einschieberichtung auf gleicher Höhe wie der Anschlag 50, d.h. die Klaue 22 befindet und noch nicht bis auf Höhe der Hammerkopfaufnahme 24 verschoben worden ist.

Wie ersichtlich sind die Hammerköpfe 28 rotationssymmetrisch ausgebildet. So können die Pumpkolben 30 frei drehbar an dem Pumpengehäuse 12 gelagert werden. Dem gegenüber sind die Antriebsstößel 18 verdrehfest in dem Antriebsgehäuse 2 montiert, sodass die C-förmige Öffnung der Klauen 22 so ausgerichtet ist, dass die Schwenkbewegung des Pumpenmoduls 10 in die Endposition aufgrund der exzentrischen Anordnung der Antriebsstößel 18 und der Pumpkolben 30 relativ zu der Schwenkachse dieser Schwenkbewegung, jedoch mit gleichem Radius um diesen Mittelpunkt zu einer zwangsläufigen Aufnahme der Zylinderabschnitte 32 der entsprechenden Pumpkolben 30 führt.

Wie sich aufgrund der obigen allgemeinen Beschreibung ergibt, hat die Antriebseinheit eine rotationssymmetrische Ausnehmung zur Aufnahme eines ebenfalls rotationssymmetrisch ausgebildeten zylindrischen Abschnitts, der bei dem konkreten Ausführungsbeispiel durch die Hülse 44 gebildet wird. Dieser Abschnitt 44 ist im Grunde nach Art einer Passung innerhalb der Ausnehmung 8 aufgenommen und damit gehalten. Lediglich die Formschlusselemente 16 bzw. Formschlussgegenelemente 26, 36 über- bzw. hinter ragen die zylindrische Fläche. So ist das Pumpennnmodul 10 innerhalb der Ausnehmung 8 schwenkbar geführt und gelagert. Die Schwenkachse dieser Schwenkbewegung bildet dabei die Mittellängsachse der Ausnehmung 8 bzw. die Mittellängsachse des zylindrischen Abschnitts 44 aus. Durch die exzentrische Anordnung des Antriebselementes 18 bzw. Antriebsgegenelementes 30 relativ zu dieser Mittellängsachse und die relative Anordnung des Antriebselementes 18 relativ zu dem Antriebsgegenelement 30 nach Erreichen der axialen Endlage und vor dem Schwenken zur formschlüssigen Verriegelung des Pumpenmoduls 10 an dem Antriebsgehäuse 2 bewirkt eine zwangsläufige formflüssige Kopplung zwischen dem Antriebselement 18 und dem Antriebsgegenelement 30 im Rahmen der Schwenkbewegung. Dabei bildet die Klaue 22 des als Antriebsmittel ausgebildeten Pumpkolbens 30 das Formschlussmittel der Antriebseinheit aus. Das hierzu vorgesehene Formschlussgegenmittel wird bei dem gezeigten Ausführungsbeispiel durch den Hammerkopf 28 gebildet.

### Bezugszeichenliste

- 1: Antriebseinheit
- 2: Antriebsgehäuse
- 4: Halterung
- 6: Bedienelement
- 8: Ausnehmung
- 10: Pumpenmodul
- 12: Pumpengehäuse
- 14: Federarm16 Nocken
- 18: Antriebsstößel
- 20: Anschlagfläche
- 22: Klaue
- 24: Hammerkopfaufnahme
- 25: Dichtung
- 26: Nut
- 28: Hammerkopf
- 30: Pumpkolben
- 32: Zylinderabschnitt
- 34: Gegenfläche
- 36: Quernut
- 37: Rast- und Schaltvorsprung
- 38: Teilungsebene
- 39: Schalter
- 40: Richtungspfeil
- 41: Gegenmarkierung
- 42: Pumpenausnehmung
- 44: Hülsenabschnitt
- 50: Anschlag

## Patentansprüche

1. Vorrichtung zur Erzeugung eines Flüssigkeitsstrahls, insbesondere für die Entfernung von biologischem Gewebe, mit einem in einem Antriebsgehäuse (2) vorgesehenen Antrieb und einem ein Pumpengehäuse (12) aufweisenden Pumpenmodul (10), wobei das Antriebsgehäuse (2) und das Pumpenmodul (10) lösbar fügbar sind, wobei das Antriebsgehäuse (2) zumindest ein Formschlusselement (16) und das Pumpengehäuse (12) zumindest ein Formschlussgegenelement (26, 36) aufweist, **dadurch gekennzeichnet, dass** das Formschlusselement (16) beim Fügen von Pumpen- und Antriebsgehäuse (12; 2) derart mit dem Formschlussgegenelement (26, 36) zusammenwirkt, das dem Pumpengehäuse (12) bis zum Erreichen einer Endposition, in der das Pumpengehäuse (12) mit dem Antriebsgehäuse (2) verbunden ist und zumindest ein an dem Antriebsgehäuse (2) vorgesehenes Antriebselement (18) des Antriebs mit einem zugeordneten Antriebsgegenelement (30) des Pumpenmoduls (10) verbunden ist, eine Schwenkbewegung relativ zu dem Antriebsgehäuse (2) aufgeprägt wird, wobei das Antriebselement (18) und das Antriebsgegenelement (30) exzentrisch zu einer Schwenkachse dieser Schwenkbewegung angeordnet und so ausgebildet sind, dass sich aufgrund der Schwenkbewegung eine formschlüssige Verbindung zwischen dem Antriebselement (18) und dem Antriebsgegenelement (30) ergibt.

2. Vorrichtung zur Erzeugung des Flüssigkeitsstrahls nach Anspruch 1, **dadurch gekennzeichnet, dass** das Formschlusselement (16) und das Formschlussgegenelement (26, 36) einen Bajonettverschluss ausbilden, der dem Pumpengehäuse (12) relativ zu dem Antriebsgehäuse (2) zunächst eine axiale Bewegung relativ zu einer im Wesentlichen zylindrischen Ausnehmung (8) und danach eine sich hierzu im Wesentlichen rechtwinklig erstreckende Schwenkbewegung aufdrängt.

3. Vorrichtung zur Erzeugung des Flüssigkeitsstrahls nach Anspruch 1 oder 2 **dadurch gekennzeichnet, dass** das Antriebsgehäuse (2) eine Ausnehmung (8) ausbildet, in der das Antriebselement (18) frei liegt.

4. Vorrichtung zur Erzeugung des Flüssigkeitsstrahls nach einem vorherigen Ansprüche **dadurch gekennzeichnet, dass** das Pumpengehäuse (12) eine Pumpenausnehmung (42) ausbildet, in der das Antriebsgegenelement (30) frei liegt und die von einem Hülsenabschnitt (44) des Pumpengehäuses (12) umgeben ist, das das Formschlussgegenelement (16, 26) zumindest teilweise ausbildet.

5. Vorrichtung zur Erzeugung des Flüssigkeitsstrahls nach Anspruch 3 oder 4, **dadurch gekennzeichnet, dass** die Ausnehmung (8) und/oder das Pumpengehäuse (12) zumindest teilweise rotationssymmetrisch ausgebildet ist/sind.

6. Vorrichtung zur Erzeugung des Flüssigkeitsstrahls nach einem der vorherigen Ansprüche **dadurch gekennzeichnet, dass** das Formschlusselement (16) und das Formschlussgegenelement (26, 36) eine Führung ausbilden, durch die eine eindeutige Ausrichtung des Pumpengehäuses (12) relativ zu dem Antriebsgehäuse (2) beim Fügen vorgegeben ist.

7. Vorrichtung zur Erzeugung des Flüssigkeitsstrahls nach einem der vorherigen Ansprüche **dadurch gekennzeichnet, dass** das Formschlusselement (16) und das Formschlussgegenelement (26, 36) so ausgebildet sind, dass das Pumpengehäuse (12) beim Fügen und am Ende der axialen Bewegung relativ zu dem Antriebsgehäuse (2) um einen Winkel von zwischen 10° bis 90°, bevorzugt von zwischen 30° +/- 5° schwenkbar ist.

8. Vorrichtung zur Erzeugung des Flüssigkeitsstrahls nach einem der vorherigen Ansprüche **dadurch gekennzeichnet, dass** zumindest einer von Antriebselement (18) und Antriebsgegenelement (30) axial verschieblich angeordnet ist und dass das Antriebselement (18) und das Antriebsgegenelement (30) so ausgebildet sind, dass das Antriebselement (18) mit einer Anschlagfläche (20) gegen eine Gegenfläche (34) des Antriebsgegenelementes (30) stößt, bevor die Endposition erreicht ist.

9. Vorrichtung zur Erzeugung des Flüssigkeitsstrahls nach Anspruch 7 oder 8 **dadurch gekennzeichnet, dass** das Antriebselement durch einen Antriebsstößel (18) gebildet ist, der mit seiner Anschlagfläche (20) gegen eine Gegenfläche (34) eines das Antriebsgegenelement ausbildenden Pumpkolben (30) anstößt, bevor das Fügen von Antriebsgehäuse (2) und Pumpenmodul (10) in axialer Richtung beendet ist.

10. Vorrichtung zur Erzeugung des Flüssigkeitsstrahls nach einem der vorherigen Ansprüche **dadurch gekennzeichnet, dass** einer von Antriebselement (18) und Antriebgegenelement (30) einen Hammerkopf (28) ausbildet und der andere von Antriebselement (18) und Antriebsgegenelement (30) eine zum Übergreifen des Hammerkopfes (28) angepasst ausgebildete Hammerkopfaufnahme (24) ausbildet.

11. Vorrichtung zu Erzeugung des Flüssigkeitsstrahls nach Anspruch 10 **dadurch gekennzeichnet, dass** das die Hammerkopfaufnahme (24) ausbildende Element (18) verdrehfest montiert ist.

12. Vorrichtung zur Erzeugung des Flüssigkeitsstrahls nach einem der vorherigen Ansprüche **dadurch gekennzeichnet, dass** die Hammerkopfaufnahme (24) eine den Hammerkopf (18) übergreifende Klaue (22) aufweist, die den Hammerkopf (28) in der Endposition axial übergreift und vor Erreichen der Endposition einen radialen Anschlag (50) für den auf gleicher Höhe befindlichen Hammerkopf (28) derart ausbildet, dass durch das Zusammenwirken von Anschlag (50) und Hammerkopf (28) ein schwenken in die Endposition verhindert wird.

13. Antriebseinheit (1) einer Vorrichtung zur Erzeugung eines Flüssigkeitsstrahls, insbesondere für die Entfernung von biologischem Gewebe nach einem der vorherigen Ansprüche, mit einem Antrieb, der in einem Antriebsgehäuse (2) vorgesehenen ist, das zumindest ein Formschlusselement (16) aufweist, mit welchem ein Pumpenmodul (10) an dem Antriebsgehäuse (2) festlegbar ist, **dadurch gekennzeichnet, dass** an dem Antriebsgehäuse (2) eine Ausnehmung (8) vorgesehen ist, in der ein Antriebselement (18) des Antriebs freiliegt, das exzentrisch zu einer Mittellängsachse der Ausnehmung (8) angeordnet ist und das ein Formschlussmittel (22) zur formschlüssigen Koppelung des Antriebselementes (18) mit einem Antriebsgegenelement (30) durch Schwenken des Pumpenmoduls (10) um die Mittellängsachse der Ausnehmung (8) zur formschlüssigen Festlegung des Pumpenmoduls (10) an dem Antriebsgehäuse (2) aufweist.

14. Pumpenmodul (10) einer Vorrichtung zur Erzeugung eines Flüssigkeitsstrahls, insbesondere für die Entfernung von biologischem Gewebe nach einem der vorherigen Ansprüche, mit einem Pumpengehäuse (12), **dadurch gekennzeichnet, dass** das Pumpengehäuse (12) zumindest ein Formschlussgegenelement (26, 36), mit welchem das Pumpenmodul (10) an dem Antriebsgehäuse (2) eines Antriebs festlegbar ist, und einen zum Einbringen in eine Ausnehmung (8) des Antriebsgehäuses (2) angepasst ausgebildeten zylindrischen Abschnitt (44) aufweist, und dass ein Antriebsgegenelement (30) vorgesehen ist, das exzentrisch zu einer Mittellängsachse des zylindrischen Abschnitts (44) angeordnet ist und ein Formschlussgegenmittel (28) zur formschlüssigen Koppelung des Antriebsgegenelementes (30) mit einem in der Ausnehmung freiliegenden Antriebselement (18) durch Schwenken des Pumpenmoduls um die Mittellängsachse des zylindrischen Abschnitts (44) zur formschlüssigen Festlegung des Pumpenmoduls (10) an dem Antriebsgehäuse (2) aufweist.

## Claims

1. Device for producing a fluid jet, in particular for the removal of biological tissue, with a drive provided in a drive casing (2) and a pump module (10) comprising a pump casing (12), where said drive casing (2) and said pump module (10) can be detachably joined, where said drive casing (2) comprises at least one positive-locking element (16) and said pump casing (12) at least one positive-locking counter-element (26, 36),
**characterized in that**
said positive-locking element (16) when joining said pump and said drive casing (12, 2) interacts with said positive-locking counter-element (26, 36) such that said pump casing (12) is, until a final position has been reached in which said pump casing (12) is connected to said drive casing (2) and at least one drive element (18) of the drive provided at said drive casing (2) is connected to an associated drive counter-element (30) of said pump module (10), imposed a pivotal motion relative to said drive casing (2), where said drive element (18) and said drive counter-element (30) are arranged eccentric to a pivot axis of said pivotal motion and configured such that a positive-locking connection between said drive element (18) and said drive counter-element (30) arises due to the pivotal motion.

2. Device for producing said fluid jet according to claim 1, **characterized in that** said positive-locking element (16) and said positive-locking counter-element (26, 36) form a bayonet lock which imposes upon said pump casing (12) relative to said drive casing (2) first an axial motion relative to a substantially cylindrical recess (8) and thereafter a pivotal motion extending substantially perpendicular thereto.

3. Device for producing said fluid jet according to claim 1 or 2, **characterized in that** said drive casing (2) forms a recess (8) in which said drive element (18) is exposed.

4. Device for producing said fluid jet according to one of the preceding claims
**characterized in that** said pump casing (12) forms a pump recess (42) in which said drive counter-element (30) is exposed and which is enclosed by a sleeve section (44) of said pump casing (12) which at least in part forms said positive-locking counter-element (16,26).

5. Device for producing said fluid jet according to claim 3 or 4, **characterized in that** said recess (8) and/or said pump casing (12) is/are at least in part configured to be rotationally symmetrical.

6. Device for producing said fluid jet according to one of the preceding claims
**characterized in that** said positive-locking element (16) and said positive-locking counter-element (26, 36) form a guide with which a unique orientation of said pump casing (12) relative to said drive casing (2) is defined during joining.

7. Device for producing said fluid jet according to one of the preceding claims
**characterized in that** said positive-locking element (16) and said positive-locking counter-element (26, 36) are configured such that said pump casing (12) is during joining and at the end of the axial motion relative to said drive casing (2) pivotable by an angle of between 10° to 90°, preferably of between 30° +/- 5°.

8. Device for producing said fluid jet according to one of the preceding claims
**characterized in that** at least one of said drive element (18) and said drive counter-element (30) is arranged axially displaceable and that said drive element (18) and said drive counter-element (30) are configured such that said drive element (18) with an abutment surface (20) abuts against a counter-surface (34) of said drive counter-element (30) before the final position has been reached.

9. Device for producing said fluid jet according to claim 7 or 8, **characterized in that** said drive element is formed by a drive pusher (18) which with its abutment surface (20) abuts against a counter-surface (34) of a pump piston (30) forming said drive counter-element, before joining of said drive casing (2) and said pump module (10) in the axial direction is completed.

10. Device for producing said fluid jet according to one of the preceding claims
**characterized in that** one of a drive element (18) and a drive counter-element (30) forms a hammerhead (28) and the other of said drive element (18) and said drive counter-element (30) forms a hammerhead seat (24) formed adapted to engage over said hammerhead (28).

11. Device for producing said fluid jet according to claim 10, **characterized in that** said element (18) forming said hammerhead seat (24) is mounted in a rotationally-fixed manner.

12. Device for producing said fluid jet according to one of the preceding claims **characterized in that** said hammerhead seat (24) comprises a claw (22) engaging over said hammer head (18) and in the final position engaging axially over said hammer head (28) and before the final position has been reached forming a radial stop (50) for said hammerhead (28) located at the same level such that pivoting to the final position is prevented by the interaction of said stop (50) and said hammerhead (28).

13. Drive unit (1) of a device for producing a fluid jet, in particular for the removal of biological tissue according to one of the preceding claims, with a drive being provided in a drive casing (2) comprising at least one positive-locking element (16) with which a pump module (10) can be fixed to said drive casing (2), **characterized in that** the drive casing (2) is provided with a recess (8) in which a drive element (18) of said drive is exposed which is arranged eccentric relative to a center longitudinal axis of said recess (8) and which comprises a positive-locking mechanism (22) for coupling said drive element (18) in a positive-locking manner to a drive counter-element (30) by pivoting said pump module (10) about the center longitudinal axis of said recess (8) for fixing said pump module (10) at said drive casing (2) in a positive-locking manner.

14. Pump module (10) of a device for producing a fluid jet, in particular for the removal of biological tissue according to one of the preceding claims, with a pump casing (12), **characterized in that** said pump casing (12) comprises at least one positive-locking counter-element (26, 36) with which said pump module (10) can be fixed to said drive casing (2) of a drive, and a cylindrical section (44) formed adapted for being introduced into a recess (8) of said drive casing (2), and that a drive counter-element (30) is provided which is arranged eccentric relative to a center longitudinal axis of said cylindrical section (44) and has a positive-locking counter-mechanism (28) for coupling said drive counter-element (30) in a positive-locking manner to a drive element (18) exposed in said recess by pivoting said pump module around the center longitudinal axis of said cylindrical section (44) for fixing said pump module (10) at said drive casing (2) in a positive-locking manner.

## Revendications

1. Dispositif destiné à la production d'un jet de liquide, en particulier pour l'élimination de tissu biologique, muni d'un entraînement installé dans un boîtier d'entraînement (2) et d'un module de pompe (10) présentant un boîtier de pompe (12), dans lequel le boîtier d'entraînement et le module de pompe (10) peuvent être reliés de manière amovible, dans lequel le boîtier d'entraînement (2) présente au moins un élément à ajustement de forme (16) et le boîtier de pompe (12) au moins un contre-élément à ajustement de forme (26, 36), **caractérisé en ce que** l'élément à ajustement de forme (16) coopère lors de l'assemblage du boîtier de pompe et du boîtier d'entraînement (12 ; 2) de telle sorte avec le contre-élément à ajustement de forme (26, 36) qu'un mouvement de pivotement par rapport au boîtier d'entraînement (2) est imprimé au boîtier de pompe (12) jusqu'à ce qu'il atteigne une position finale, dans laquelle le boîtier de pompe (12) est relié au boîtier d'entraînement (2) et au moins un élément d'entraînement (18) prévu dans le boîtier d'entraînement (2) est relié à un contre-élément d'entraînement (30) correspondant du module de pompe (10), dans lequel l'élément d'entraînement (18) et le contre-élément d'entraînement (30) sont excentrés par rapport à un axe de pivotement de ce mouvement de pivotement et conçues de telle sorte que l'on obtient une liaison clabotée entre l'élément d'entraînement (18) et le contre-élément d'entraînement (30) grâce au mouvement de pivotement.

2. Dispositif destiné à la production d'un jet de liquide selon la revendication 1, **caractérisé en ce que** l'élément à ajustement de forme (16) et le élément à ajustement de forme (26, 36) constituent une fermeture à baïonnette, qui imprime au boîtier de pompe (12) par rapport au boîtier d'entraînement (2) d'abord un mouvement axial par rapport à un évidement sensiblement cylindrique (8) et ensuite un mouvement de pivotement s'étendant sensiblement à angle droit.

3. Dispositif destiné à la production d'un jet de liquide selon les revendications 1 ou 2, **caractérisé en ce que** le boîtier d'entraînement (2) constitue un évidement (8) dans lequel l'élément d'entraînement (18) repose librement.

4. Dispositif destiné à la production d'un jet de liquide selon l'une des revendications précédentes, **caractérisé en ce que** le boîtier de pompe (12) constitue un évidement de pompe (42) dans lequel le contre-élément d'entraînement (30) repose librement et qui est entouré d'une partie manchon (44) du boîtier de pompe (12) constituant au moins partiellement le contre-élément à ajustement de forme (16, 26).

5. Dispositif destiné à la production d'un jet de liquide selon les revendications 3 ou 4,
**caractérisé en ce que** l'évidement (8) et/ou le boîtier de pompe (121) sont au moins partiellement conçus symétriques en rotation.

6. Dispositif destiné à la production d'un jet de liquide selon l'une des revendications précédentes, **caractérisé en ce que** l'élément à ajustement de forme (16) et le contre-élément à ajustement de forme (26, 36) constituent un guidage, à travers lequel est prévu une orientation univoque du boîtier de pompe (12) par rapport au boîtier d'entraînement (2) lors d'assemblage.

7. Dispositif destiné à la production d'un jet de liquide selon l'une des revendications précédentes, **caractérisé en ce que** l'élément à ajustement de forme (16) et le contre-élément à ajustement de forme (26, 36) sont conçus de telle sorte que le boîtier de pompe (12) lors de l'assemblage et au terme du mouvement axial par rapport au boîtier d'entraînement (2) est pivotable d'un angle compris entre 10° et 90°, de préférence de l'ordre de 30° +/- 5°.

8. Dispositif destiné à la production d'un jet de liquide selon l'une des revendications précédentes, **caractérisé en ce qu'**au moins l'un de l'élément d'entraînement (18) et du contre-élément d'entraînement (30) est mobile dans l'axe et que l'élément d'entraînement (18) et le contre-élément d'entraînement (30) sont conçus de telle sorte que l'élément d'entraînement (18) bute avec une surface de butée (20) contre une contre-surface (34) du contre-élément d'entraînement avant d'atteindre la position finale.

9. Dispositif destiné à la production d'un jet de liquide selon les revendications 7 ou 8,
**caractérisé en ce que** l'élément d'entraînement est constitué d'un poussoir d'entraînement (18) qui bute avec sa surface de butée (20) contre une contre-surface (34) d'un piston de pompe (30) constituant le contre-élément d'entraînement, avant le terme de l'assemblage du boîtier d'entraînement (2) et du module de pompe (10) dans la direction axiale.

10. Dispositif destiné à la production d'un jet de liquide selon l'une des revendications précédentes, **caractérisé en ce que** l'un de l'élément d'entraînement (18) et du contre-élément d'entraînement (30) forme une tête de marteau (28) et que l'autre de l'élément d'entraînement (18) et du contre-élément d'entraînement (30) forme un réceptacle (24) de tête de marteau, prévu pour mettre en prise la tête de marteau (28).

11. Dispositif destiné à la production d'un jet de liquide selon la revendication 10 précédentes, **caractérisé en ce que** l'élément (18) constituant le réceptacle de tête de marteau (24) est monté fixe en rotation.

12. Dispositif destiné à la production d'un jet de liquide selon l'une des revendications précédentes, **caractérisé en ce que** le réceptacle (24) de tête de marteau présente un ergot (22) mettant en prise la tête de marteau (18), qui met la tête de marteau (28) en prise axialement dans la position finale et avant d'atteindre la position finale forme une butée radiale (50) pour la tête de marteau (28) située à la même hauteur de telle sorte que la coopération de la butée (50) et de la tête de marteau (28) empêche tout pivotement dans la position finale.

13. Unité d'entraînement (1) d'un dispositif destiné à la production d'un jet de liquide, en particulier pour l'élimination de tissu biologique, selon l'une des revendications précédentes, muni d'un entraînement installé dans un boîtier d'entraînement (2), qui présente au moins un élément à ajustement de forme (16), avec lequel un module de pompe (10) peut être fixé au boîtier d'entraînement (2), **caractérisé en ce qu'**un évidement est prévu sur le boîtier d'entraînement (2), réceptacle dans lequel un élément d'entraînement (18) de l'entraînement repose librement, de manière excentrée par rapport à un axe longitudinal médian de l'évidement (8) et qui présente un moyen à ajustement de forme (22) pour l'accouplement par concordance des formes de l'élément d'entraînement (18) avec un contre-élément d'entraînement (30) par pivotement du module de pompe (10) autour de l'axe longitudinal médian de l'évidement (8) pour fixer le module de pompe (10) sur le boîtier d'entraînement (2) par concordance des formes.

14. Module de pompe (10) d'un dispositif destiné à la production d'un jet de liquide, en particulier pour l'élimination de tissu biologique, selon l'une des revendications précédentes, muni d'un entraînement installé dans un boîtier de pompe (12), **caractérisé en ce que** le boîtier de pompe (12) présente au moins un contre-élément à ajustement de forme (26, 36), avec lequel le module de pompe (10) peut être fixé au boîtier d'entraînement (2) d'un entraînement, et une section cylindrique prévu pour venir dans un évidement (8) du boîtier d'entraînement (2), et qu'un contre-élément d'entraînement (30) est prévu excentré par rapport à un axe longitudinal médian de la section cylindrique (44) et qui présente un contre-moyen à ajustement de forme (28) pour l'accouplement par concordance des formes du contre-élément d'entraînement (30) avec un élément d'entraînement (18) reposant librement dans l'évidement par pivotement du module de pompe autour de l'axe longitudinal médian de la section cylindrique (44) pour fixer le module de pompe (10) sur le boîtier d'entraînement (2) par concordance des formes.
